# FASCICULE DE BREVET EUROPEEN

(11) **EP 1 871 394 B1**
(45) Date de publication et mention de la délivrance du brevet: **17.11.2010**
(21) Numéro de dépôt: 06726178.4
(22) Date de dépôt: 04.04.2006
(51) Int. Cl.: A61K 31/718

(54) **COMPOSITION ANTI-INFLAMMATOIRE ET/OU ANALGESIQUE POUR L`INTESTIN COMPRENANT DES MALTODEXTRINES BRANCHEES**
ENTZÜNDUNGSHEMMENDE UND/ODER ANALGETISCHE ZUSAMMENSETZUNG FÜR DEN DARM MIT VERZWEIGTEN MALTODEXTRINEN
ANTI-INFLAMMATORY AND/OR ANALGESIC COMPOSITION FOR THE INTESTINE COMPRISING BRANCHED MALTODEXTRINS

(30) Priorité: 18.04.2005 FR 0503852
(43) Date de publication de la demande: 02.01.2008
(73) Titulaire: ROQUETTE FRERES, 62136 Lestrem (FR)
(72) Inventeur: WILS, Daniel, F-59190 Morbecque (FR); DEREMAUX, Laëtitia, F-59800 Lille (FR); SANIEZ, Marie-Hélène, F-59800 Lille (FR)
(74) Mandataire: Cabinet Plasseraud
(86) Numéro de dépôt international: PCT/FR2006/000736
(87) Numéro de publication internationale: WO 2006/111624

(56) Documents cités:
- EP-A- 1 245 578
- GB-A- 2 363 713
- US-A1- 2002 182 299
- US-A1- 2003 039 740
- US-A1- 2004 030 151
- US-A1- 2004 058 055
- US-B1- 6 630 586
- FRENCH M A ET AL: "Polyunsaturated fat in the diet may improve intestinal function in patients with Crohn's disease" BIOCHIMICA ET BIOPHYSICA ACTA. MOLECULAR BASIS OF DISEASE, AMSTERDAM, NL, vol. 1360, no. 3, 24 mai 1997 (1997-05-24), pages 262-270, XP004277537 ISSN: 0925-4439

## Description

L'invention concerne une composition anti-inflammatoire et/ou analgésique de l'intestin, enrichie en fibres, **caractérisée en ce qu**'elle comprend des maltodextrines branchées.

Les Maladies Inflammatoires Chroniques de l'Intestin (ou MICI) regroupent notamment deux affections distinctes : la Recto-Colite ulcéro-Hémorragique (RCH) et la maladie de Crohn. Ces deux maladies, tout à la fois distinctes et apparentées, sont caractérisées par des lésions inflammatoires plus ou moins diffuses de l'intestin, notamment dues à un état d'hyperactivation du système immunitaire de l'intestin dont l'origine est inconnue.

Leur expression est essentiellement digestive avec de la diarrhée, des douleurs abdominales, un amaigrissement, une inflammation des tissus.

La fréquence des MICI est en progression depuis les dernières décennies. Ceci s'explique en partie par les progrès techniques réalisés qui permettent de diagnostiquer plus facilement les maladies, mais il apparaît surtout que le changement des habitudes alimentaires intervient dans l'évolution de ces maladies, tout comme les allergies alimentaires, l'obésité et autres « maladies de civilisation ».

Depuis quelque temps, un intérêt considérable s'est porté à l'association de régimes alimentaires adaptés aux traitements thérapeutiques plus classiques.

Les prébiotiques et les probiotiques sont plus particulièrement étudiés pour entrer dans la diététique des personnes touchées par ces pathologies, permettant d'améliorer leurs conditions de vie et de jouer un rôle important quant à l'aspect préventif de ces troubles.

Plus particulièrement, il est reconnu que l'apport de fibres appropriées dans l'alimentation a un effet bénéfique sur la santé, ces fibres exerçant un effet protecteur dans les inflammations du côlon.

Ces fibres sont généralement classées en deux catégories : les fibres solubles et les fibres insolubles.

Les fibres solubles, comme la pectine et l'inuline, non digestibles par les enzymes de l'homme, sont fermentées par la flore bactérienne intestinale. Cette fermentation libère des acides gras à courte chaîne dans le côlon, qui ont pour effet de diminuer le pH de celui-ci et par voie de conséquence de limiter le développement de bactéries pathogènes.

Les fibres insolubles, comme la cellulose, les amidons résistants, les fibres de mais (drêche) ou de soja, ont un rôle essentiellement mécanique dans le tractus gastro-intestinal. Elles ne sont que très peu fermentées par la flore intestinale et contribuent à la réduction du temps de transit intestinal par effet de lest.

Il résulte des nombreuses études tendant à démontrer l'importance du régime alimentaire dans la prévention de l'inflammation du côlon, qu'il existe une relation entre les sucres complexes (polysaccharides, amidon) et la bonne physiologie du côlon.

Ce sont notamment les amidons résistants, non digérés dans l'intestin grêle, qui présentent un grand intérêt pour la santé du côlon.

Cependant, il reste encore beaucoup de travail à accomplir, de manière à modifier la composition des aliments en amidons résistants sans en changer les propriétés organoleptiques.

Depuis 1997, KANAUCHI et al décrivent les effets de denrées alimentaires à base d'orge germée sur des colites ou des diarrhées induites chez les animaux de laboratoire (in Biosci. Biotech. Biochem. 1997, 61, 449-454 et in J. Gastroenterol. 1998, 33, 179-188).

Mais depuis ces dernières années, les spécialistes de ces pathologies se tournent plutôt vers les « aliments coliques », et plus particulièrement les prébiotiques.

Ces prébiotiques sont définis comme des fertilisants de bactéries bénéfiques pour la santé qui colonisent le côlon.

Les prébiotiques sont des ingrédients fonctionnels présents dans de nombreuses plantes comestibles et dans de nombreux produits alimentaires.

Les composés classiquement classés dans les prébiotiques sont les fructooligosaccharides et les transgalacto-oligosaccharides, mais également le lactulose, les isomaltooligosaccharides, les oligosaccharides extraits du soja, les xylooligosaccharides...

Les cibles de leurs effets fonctionnels sont la flore colique qui les fermentent et pour laquelle ils servent de substrats spécifiques et sélectifs, la physiologie gastro-intestinale et en particulier les fonctions assurées par le gros intestin, le système immunitaire, la biodisponibilité des minéraux et le métabolisme des lipides.

Parmi la flore colique bénéfique dont la croissance est favorisée par les prébiotiques, sont surtout cités les bifidobactéries et les lactobacilles.

Les lactobacilles présentent l'avantage de conduire à un abaissement du pH du milieu par production d'acide lactique, cet abaissement du pH empêchant la croissance de flores pathogènes telles les protéobactéries ou les entérobactéries, agents causals de pathologies comme la maladie de Crohn ou certaines colites ulcérantes.

Les bifidobactéries sont notamment décrites pour leur production d'activités enzymatiques de type glucosidases, qui favorisent la libération de flavonoides à effets anti-mutagène et anti-oxydant.

Les maladies inflammatoires et leur traitement respectif font l'objet de recherches actives. Des modèles expérimentaux d'induction de colites ont été élaborés, telle l'induction de colites par l'administration d'une solution contenant un allergène (TriNitroBenzène Sulfonate ou TNBS) dans de l'éthanol chez le rat ou la souris de laboratoire.

L'éthanol permet la destruction de la barrière constituée par la muqueuse intestinale et favorise ainsi la pénétration du TNBS dans la paroi intestinale, TNBS qui entraîne des nécroses aiguës, souvent transmurales, probablement dues à des dommages oxydatifs.

Ce modèle est envisagé pour des études d'hypersensibilité localisée du côlon, et est particulièrement approprié par le fait que les inflammations provoquées par ce modèle sont sensibles aux médicaments administrés dans le cadre des MICI.

Plusieurs compositions anti-inflammatoires de l'intestin ont été proposées et testées grâce à ce modèle animal.

Les fructooligosaccharides (FOS) sont des polymères d'unités fructose à courtes chaînes qui ne sont pas hydrolysés dans l'intestin grêle chez l'Homme, mais sont dégradés par la flore résidente du côlon.

Les FOS induisent principalement la croissance des lactobacilles et des bifidobactéries endogènes de l'intestin chez l'Homme et les animaux.

De plus, la fermentation des FOS induit une baisse du pH du côlon, induit la production d'acides gras volatils, des lactates, et accessoirement augmente la production de butyrates.

Dans leur revue publiée en 2003 dans American Society for Nutritional Sciences, vol 133, 21-27, C. CHERBUT et al décrivent l'effet préventif des FOS dans l'inflammation intestinale induite par le TNBS chez le rat de laboratoire.

Les effets protecteurs des FOS sont mesurés par le suivi du score macroscopique d'endommagement du colon (recherche visuelle des nécroses et des ulcères provoqués par le TNBS) et la mesure de l'activité myéloperoxidase (enzyme spécifique des granules neutrophiles polynucléaires, marqueur de l'inflammation intestinale).

Il est ainsi montré dans cet article que les FOS réduisent de manière significative l'inflammation intestinale, permettant de limiter les dommages intestinaux (nécroses et ulcères), diminuent l'activité myéloperoxidase, et diminuent également la perte de poids induite par le TNBS.

Par ailleurs, C. CHERBUT et al démontrent également que les FOS ont bien un effet prébiotique, i.e. sont capables de stimuler la croissance intestinale de bactéries bénéfiques dans le côlon, en l'occurrence les bactéries lactiques et butyriques, ce qui conduit à un abaissement du pH du côlon.

Le mécanisme de protection des FOS n'est pas clairement expliqué.

Il a été proposé dans la demande de brevet US 2004/0219157 que les FOS stimuleraient l'homéostasie de paramètres immunologiques non spécifiques et stimuleraient la croissance de sous-populations de lymphocytes.

Il est également supposé que les bactéries lactiques, dont la croissance est stimulée par les FOS, sont des antagonistes des bactéries pathogènes dont elles bloquent le développement par la production de substances antimicrobiennes et par abaissement du pH du côlon.

Les bactéries lactiques peuvent également adhérer aux parois intestinales et empêcher ainsi la colonisation par ces mêmes bactéries pathogènes.

Par ailleurs, les FOS agissent également sur l'abaissement du pH du côlon par la production induite des acides lactique et butyrique.

Cependant, cet effet d'acidose intestinale ne présente pas que des avantages.

Dans la demande de brevet internationale WO 04/026316, il est en effet décrit que cette acidose, particulièrement favorisée par la croissance des bactéries lactiques, finit par provoquer une érosion de la muqueuse colique, augmentant le risque de colites ulcérantes.

Par ailleurs, l'accumulation de l'acide lactique dans le côlon peut également avoir pour conséquence le relargage des quantités excédentaires dans le sang, provoquant ainsi une acidose métabolique.

L'inuline, mais également les FOS, ont l'inconvénient d'être fermentés trop rapidement dans le côlon, et peuvent ainsi conduire à des déséquilibres dans la population microbienne qui nuisent à leur effet protecteur du côlon.

Pour contrebalancer cet effet néfaste, il est proposé dans ladite demande de brevet WO 04/026316 d'associer aux FOS un polysaccharide caractérisé par sa lente fermentation dans le côlon, en l'occurrence ici le polydextrose.

Le polydextrose est synthétisé par polymérisation aléatoire du glucose en présence de sorbitol, d'un catalyseur acide adéquat (tel l'acide citrique) et à haute température.

Le polydextrose est largement utilisé dans l'alimentation comme agent de charge et comme ingrédient à faible caloricité. Le polydextrose n'est pas digéré ni absorbé dans l'intestin grêle et une partie importante est retrouvée dans les fèces.

Ce que la demande de brevet WO 04/026316 enseigne surtout, c'est l'utilisation du polydextrose pour empêcher les effets d'acidose induits par les déséquilibres provoqués dans la population microbienne du côlon, notamment par ceux induits par des agents prébiotiques tels que l'inuline et les FOS.

Le polydextrose favoriserait ainsi la consommation d'acide lactique par des flores spécifiques, contrebalançant sa surproduction induite par les FOS.

De tout ce qui précède, il résulte qu'il n'existe pas, à la connaissance de la société Demanderesse, de composition polysaccharidique unique qui satisfasse toutes les exigences d'une composition protectrice efficace de l'intestin.

La présente invention a donc pour but de remédier aux inconvénients de l'art antérieur.

La société Demanderesse a ainsi trouvé que l'incorporation de maltodextrines branchées permettait avantageusement de concilier tous les objectifs jusqu'alors réputés inconciliables, en imaginant et élaborant, au prix de nombreuses recherches, une composition nouvelle anti-inflammatoire et/ou analgésique du côlon enrichie en fibres, qui réponde à tous les critères précités, à savoir un effet protecteur de la muqueuse colique, un abaissement modéré du pH du côlon, une production favorisée des bactéries propioniques et butyriques, et dans une moindre mesure des bactéries lactiques.

L'invention a donc pour objet des maltodextrines branchées présentant entre 15 et 35 % de liaisons glucosidiques 1 → 6, une teneur en sucres réducteurs inférieure à 20 %, un indice de polymolécularité inférieur à 5 et une masse moléculaire en nombre Mn au plus égale à 4500 g/mole pour leur utilisation dans une méthode de traitement thérapeutique du corps humain ou animal.

Par maltodextrines branchées, on entend au sens de l'invention les maltodextrines décrites dans le brevet EP 1.006.128 dont la société Demanderesse est titulaire.

Toutes les compositions de maltodextrines branchées décrites dans le brevet EP 1.006.128 sont appropriées à la préparation de compositions anti-inflammatoires et/ou analgésiques de l'intestin selon l'invention.

Selon une variante préférée, celles-ci présentent une teneur en sucres réducteurs comprise entre 2 et 5 %, et une masse moléculaire en nombre Mn comprise entre 2.000 et 3.000 g/mole.

Les maltodextrines branchées présentent un taux de fibres totales supérieur ou égal à 50 % sur sec, déterminé selon la méthode AOAC n° 2001-03 (2001).

L'invention a pour objet une composition enrichie en fibres pour le traitement thérapeutique du corps humain ou animal, **caractérisée en ce qu**'elle comprend à titre de principe actif les maltodextrines branchées.

Une composition anti-inflammatoire et/ou analgésique de l'intestin enrichie en fibres selon l'invention comprend 0,5 à 20 %, de préférence 5 à 10 % en poids sec desdites maltodextrines branchées de manière à constituer un apport de fibres et un effet protecteur du côlon suffisant.

En deçà de 0,5 % en poids de maltodextrines branchées dans la composition anti-inflammatoire et/ou analgésique de l'intestin conforme à l'invention, l'apport de fibres est insuffisant pour avoir un effet détectable.

Ces maltodextrines branchées présentent un caractère d'indigestibilité qui a pour conséquence d'empêcher leur assimilation au niveau de l'intestin grêle.

Elles présentent une source de fibres indigestibles, bénéfiques pour le métabolisme et pour l'équilibre intestinal.

Leur teneur élevée en liaisons glucosidiques 1-6 leur confère en effet des propriétés prébiotiques tout à fait particulières : il est en effet apparu que les bactéries butyrogènes, lactiques ou propioniques métabolisent ces composés hautement branchés.

Ces maltodextrines branchées favorisent également le développement des bactéries bifidogènes au détriment des bactéries indésirables, et ainsi favorisent également l'expression des activités α et β glucosidases.

La composition anti-inflammatoire et/ou analgésique de l'intestin conforme à l'invention permet de stimuler d'un facteur 2 à 10, de préférence 3 à 8 les activités enzymatiques α et β glucosidases du contenu cæcal et des selles, comme il sera exemplifié ci-après.

Il en résulté des propriétés tout à fait bénéfiques pour la santé du consommateur.

D'autre part, la consommation des maltodextrines branchées de la composition anti-inflammatoire et/ou analgésique conforme à l'invention par les microorganismes du côlon conduira à abaisser de 0,5 à 1 unité le pH du contenu caecal, intestinal et fécal, ce qui traduit une croissance équilibrée des dits microorganismes.

L'utilisation de la composition anti-inflammatoire et/ou analgésique selon l'invention permet également d'augmenter la production d'acides organiques volatiles dans le cæcum, acides organiques choisis dans le groupe constitué de l'acide acétique, butyrique et propionique, de préférence les acides propionique et butyrique.

L'effet protecteur de la muqueuse colique est démontré notamment chez l'animal après administration de TNBS et se traduit par des résultats remarquables, comme il sera exemplifié ci-après.

Les animaux continuent à s'alimenter normalement, et sont significativement protégés contre l'inflammation nécrosante induite par le TNBS, comme le démontre l'abaissement de l'activité myéloperoxydase (ou MPO), dosée dans l'épithélium intestinal.

En effet, cette activité MPO traduit l'infiltration des neutrophiles dans les phagosomes et l'espace extracellulaire, et permet de quantifier le processus d'inflammation auquel elle est directement corrélée.

La composition anti-inflammatoire et/ou analgésique de l'intestin conforme à l'invention permet alors de diminuer de 5 à 40 %, de préférence de 7 à 35 %, l'activité myéloperoxydase de l'épithélium intestinal.

Cet effet traduit de manière significative l'effet protecteur desdites compositions contre l'inflammation intestinale, permettant d'envisager la préparation de compositions anti-inflammatoires et/ou analgésiques de l'intestin améliorant le bien être des malades, tant chez l'Homme que les animaux.

Il a d'autre part été trouvé que les maltodextrines branchées selon l'invention ne généraient pas de diarrhées osmotiques et ce même à des doses importantes.

Le phénomène de diarrhées osmotiques est observé lors de la consommation de carbohydrates fermentescibles de faible poids moléculaire, tels que par exemple le lactulose et les fructooligosaccharides.

Ce phénomène se traduit par une augmentation de la teneur en eau des selles en réaction à une augmentation de l'osmolarité du contenu fécal, cette augmentation de la teneur en eau pouvant aller jusqu'à l'apparition de diarrhées. De manière surprenante et inattendue, les maltodextrines branchées conformes à l'invention ne génèrent pas ce phénomène bien qu'elles soient fermentescibles.

En Alimentation, la composition anti-inflammatoire et/ou analgésique de l'intestin conforme à l'invention peut se présenter sous une forme prête à l'emploi, ou encore sous forme de boisson, comme un jus de fruit, une soupe, ou encore sous forme de yaourts ou incorporé dans les céréales du petit déjeuner.

Ladite composition peut être par ailleurs utilisée chez l'animal et plus particulièrement chez le chat, le chien, le cochon, le lapin ou les autres animaux de rente qui sont sensibles à l'inflammation intestinale, animaux présentant une diminution de leur immunité.

Cette composition peut être également proposée pour la complémentation alimentaire des personnes souffrant des MICI mais aussi pour les personnes souffrant du syndrome de l'intestin irritable, des personnes atteintes de tourista, de douleurs abdominales dont l'étiologie est souvent méconnue.

En Pharmacie, une composition anti-inflammatoire et analgésique de l'intestin conforme à l'invention peut comprendre les maltodextrines branchées et au moins un autre principe actif, dans une proportion fonction de la nature du principe actif considéré.

De préférence cet autre principe actif est un agent anti-inflammatoire de l'intestin.

Dans le traitement des MICI, par exemple, deux types de traitements peuvent être proposés :
- un traitement à l'aide de médicaments dérivés de salicylés comme la sulfasalazine ou ses dérivés tels les 5-aminosalicylates (5-ASA),
- un traitement à base de médicaments de la famille des corticoïdes comme la cortisone ou la prednisolone.

Un mode de réalisation de l'invention concerne une composition telle que décrite précédemment comprenant les maltodextrines branchées, **caractérisée en ce qu**'elle comprend en outre au moins un principe actif choisi dans le groupe constitué par la sulfasalazine et ses dérivés et les corticoïdes.

Selon un mode particulier de réalisation de l'invention concerne une composition telle que décrite précédemment comprenant les maltodextrines branchées, comprenant en outre un principe actif choisi dans le groupe constitué par la sulfasalazine et ses dérivés, **caractérisée en ce que** le rapport poids de maltodextines branchées sur poids de sulfasalazine ou d'un de ses dérivés est compris entre 2 et 30.

Un mode particulier de réalisation de l'invention concerne une composition telle que décrite précédemment comprenant les maltodextrines branchées, comprenant en outre un principe actif choisi dans le groupe constitué par les corticoïdes, **caractérisée en ce que** le rapport poids de maltodextrines branchées sur poids de corticoïde est compris entre 2 et 250.

Typiquement une composition selon l'invention peut se présenter sous forme liquide, en poudre, en sirop, en suppositoire, en comprimé ou en tablette.

Un mode de réalisation de l'invention concerne un kit pour le traitement thérapeutique du corps humain ou animal, comprenant :
a) une première composition telle que décrite précédemment comprenant les maltodextrines branchées ; et
b) une deuxième composition comprenant un agent anti-inflammatoire de l'intestin.

Un mode de réalisation de l'invention est une méthode pour traiter ou prévenir les inflammations de l'intestin et/ou calmer les douleurs au niveau de l'intestin comprenant l'administration à un individu d'une quantité thérapeutique suffisante de maltodextrines branchées.

Un mode particulier de réalisation de l'invention est une méthode pour traiter ou prévenir les inflammations de l'intestin et/ou calmer les douleurs au niveau de l'intestin comprenant l'administration à un individu ou à un animal d'une composition telle que décrite précédemment comprenant les maltodextrines branchées.

Les compositions décrites précédemment comprenant des maltodextrines branchées pourront avantageusement être administrées à un individu ou à un animal en combinaison avec une deuxième composition comprenant un agent anti-inflammatoire de l'intestin. Lors du traitement les deux compositions pourront être administrées de manière concomitante ou de manière espacée dans le temps. Le mode d'administration de la deuxième composition est fonction de l'agent anti-inflammatoire de l'intestin utilisé.

Un mode particulier de réalisation de l'invention est une méthode pour traiter ou prévenir les inflammations de l'intestin et/ou calmer les douleurs au niveau de l'intestin comprenant l'administration concomitante ou espacée dans le temps des deux compositions décrites dans le kit décrit précédemment.

Un mode de réalisation de l'invention est l'utilisation de maltodextrines branchés pour la fabrication d'une composition ou d'un kit pour traiter ou prévenir les inflammations de l'intestin et/ou calmer les douleurs au niveau de l'intestin.

Parmi les maladies et les douleurs que l'on peut traiter ou prévenir, on peut citer la maladie inflammatoire chronique de l'intestin, le syndrome de l'intestin irritable, la tourista ou les douleurs abdominales. Parmi les maladies inflammatoires chroniques de l'intestin, on peut citer la recto-colite ulcéro-hémorragique et la maladie de Crohn.

Quant au rôle analgésique de la composition de l'invention, il est estimé en regard de l'expression des récepteurs PPARγ et MOR.

Les PPARγ (ou *« Peroxisome Proliferator Activated Receptor γ»)* font partie de la famille des récepteurs nucléaires. Ils sont notamment activés par les acides gras et sont impliqués dans la transduction des signaux métaboliques et nutritionnels en réponses transcriptionnelles. Ils jouent un rôle majeur dans le maintien de l'intégrité de la muqueuse intestinale.

Il est connu de l'homme du métier que les PPARγ sont fortement impliqués dans la régulation de l'inflammation du côlon. Ils sont également exprimés dans le cas des cancers du côlon et leur activation inhibe la croissance cellulaire et la différenciation cellulaire.

Les MOR (ou « µ Opioid Receptor ») sont trouvés dans le système nerveux central et périphérique et peuvent être présents notamment dans le côlon. La principale fonction des MOR est la fonction analgésique. La deuxième fonction est l'inhibition de la mobilité intestinale. Les MOR sont également impliqués dans la régulation de l'inflammation intestinale.

Comme il sera exemplifié ci-après, il est remarquable de constater que la composition de l'invention permet d'augmenter d'un facteur 1,2 à 3, de préférence d'un facteur 1,6 à 2 l'activité du « Peroxisome Proliferator Activated Receptor γ» (PPAR-γ).

De la même manière la composition de l'invention permet d'augmenter le nombre des « µ Opioid Receptor » (MOR) d'un facteur 1,2 à 10, de préférence d'un facteur 2,5 à 7,5, plus préférentiellement encore d'un facteur 4 à 5.

Ladite composition est enfin particulièrement adaptée aux sujets stressés dont le stress se manifeste au niveau digestif.

L'invention sera mieux comprise à la lecture des exemples qui suivent et qui se veulent illustratifs et non limitatifs.

### Exemple 1

On étudie chez le rat de laboratoire l'effet dans leur alimentation des compositions comprenant des maltodextrines branchées de l'invention (MDB) ou du glucose (témoin) associées avec des fibres insolubles (drêche de maïs ou cellulose), dans la protection de leur muqueuse colique après administration de TNBS.

L'association des fibres insolubles (en l'occurrence ici les drêches de maïs) avec les MDB de l'invention est réalisée de manière à mimer l'apport en fibres des produits céréaliers dans l'alimentation selon les recommandations des autorités sanitaires.

En outre, les drêches de maïs ont été choisies pour leur richesse en caroténoïdes et en polyphénols (notamment en acides phénolique et férulique).

Les maltodextrines branchées de l'invention choisies dans cet exemple présentent entre 15 et 35 % de liaisons glucosidiques 1→6, une teneur en sucres réducteurs comprise entre 2 et 5 %, un indice de polymolécularité inférieur à 5 et une masse moléculaire moyenne en nombre Mn comprise entre 2000 et 3000 g/mole :

| Sucres réducteurs | 2,3 |
|---|---|
| Mn (g/mole) | 2480 |
| Mw (g/mole) | 5160 |
| Liaison 1,2 (%) | 10 |
| Liaison 1,3 (%) | 12 |
| Liaison 1,4 (%) | 49 |
| Liaison 1,6 (%) | 29 |

Elles présentent en outre un taux de fibres totales de 90 % sur sec, déterminé selon la méthode AOAC (N°2001-03).

64 rats OFA d'origine Sprague Dawley sont répartis en 8 groupes qui reçoivent chacun dans leur alimentation et dans leur boisson un régime particulier dont la composition est donnée dans le tableau I suivant.

Le glucose et les maltodextrines branchées sont présents dans la boisson de la ration alimentaire à raison de 5 % en poids/poids. La cellulose et les drêches sont présents dans l'aliment de la ratio alimentaire à raison de 5 % en poids/poids.

**Tableau I**

| Groupe | Produit testé dans la boisson | Produit testé Dans l'aliment | Injection Intra-rectale |
|---|---|---|---|
| 1 | Glucose | Cellulose | NaCl |
| 2 | MDB | Cellulose | NaCl |
| 3 | Glucose | Drêche | NaCl |
| 4 | MDB | Drêche | NaCl |
| 5 | Glucose | Cellulose | TNBS |
| 6 | MDB | Cellulose | TNBS |
| 7 | Glucose | Drêche | TNBS |
| 8 | MDB | Drêche | TNBS |

Après une semaine de quarantaine au cours de laquelle les animaux reçoivent une alimentation standard et de l'eau potable, les rats consomment la nourriture et la boisson selon le régime alimentaire décrit dans le tableau 1 durant 20 jours.

Puis ils sont mis à jeun durant 24 heures.

A J₂₁, les animaux sont traités par injection intra-rectale avec les produits préconisés dans le tableau 1.

Les animaux des groupes 5 à 8 reçoivent une injection intra-rectale de 500 µl de TNBS diluée dans de l'éthanol à 40 % Gay Lussac, tandis que les groupes 1 à 4 reçoivent une injection intra-rectale de 500 µl de NaCl à 9 %o.

Le TNBS est injecté à la dose de 10 mg/kg de poids corporel et par jour.

Cette dose est connue pour produire une réaction inflammatoire sévère mais réversible.

L'évolution pondérale des animaux est suivie les 3 jours suivant l'injection.

A J₂₄, les animaux sont sacrifiés par asphyxie au CO₂.

Les animaux sont pesés, puis après autopsie, le côlon est prélevé, vidé puis pesé.

Il est ensuite observé à l'oeil nu et un score de Wallace lui est attribué.

L'activité myéloperoxydase (ou MPO) est également dosée dans l'épithélium intestinal.

Cette activité traduit l'infiltration des neutrophiles dans les phagosomes et l'espace extracellulaire, et permet de quantifier le processus d'inflammation auquel elle est directement corrélée.

Le score de Wallace est établi en utilisant l'échelle de Wallace telle que présentée dans le tableau II suivant.

**Tableau II**

| Score | Observations macroscopiques |
|---|---|
| 0 | Aucun dommage. |
| 1 | Hyperémie. Pas d'ulcère. |
| 2 | Hyperémie et épaississement de la muqueuse. Pas d'ulcère. |
| 3 | Un ulcère sans épaississement de la muqueuse. |
| 4 | 2 ou plusieurs sites d'ulcération ou d'inflammation. |
| 5 | 2 ou plusieurs sites d'ulcération ou d'inflammation ou un site d'ulcération/inflammation s'étendant sur plus d'l cm sur la longueur du côlon. |
| 6 et + | Si les dommages couvrent plus de 2 cm sur la longueur du côlon, le score est augmenté de 1 pour chaque cm lésé supplémentaire. |

Quant au dosage de l'activité MPO, il nécessite une préparation du côlon selon le protocole suivant.

On met les fragments de côlon en suspension dans 6 ml de tampon Hexa-decyl-Trimethyl Ammonium Bromide (0,5 % d'HTAB dans un tampon phosphate 50 mM, pH 7,0). On broie et homogénéise les fragments ainsi traités à l'aide d'un POLYTRON pendant 10 s. On traite chaque échantillon aux ultrasons à l'aide d'un appareil de marque VIBRA CELL 500 Watts de la société Sonics et Materials Inc. Danbury Connecticut, USA (puissance au convertisseur de 500 W, puissance dissipée à la sonde de 30 % -soit 150 W/cm², pulseur en position 2 - soit 66 % de seconde).

Les sonicats subissent ensuite 3 cycles de congélation-décongélation avant d'être à nouveau traités par ultrasons dans les mêmes conditions. On centrifuge ensuite les échantillons pendant 15 min à 10.000 g à 4°C.

On récupère le surnageant pour le dosage de la MPO. La détermination de l'activité MPO est basée sur une oxydation d'un donneur d'hydrogène artificiel dépendante du peroxyde d'hydrogène (guaïacol) qui, sous sa forme oxydée, devient orangée.

Le suivi de la densité apparente à 470 nm et à 30°C donne les valeurs d'activités (exprimées en Unités d'absorbance/minute/gramme de côlon). L'ensemble des résultats obtenus est présenté dans les tableaux III et IV suivants (valeurs exprimées comme la moyenne des résultats des mesures effectuées sur les 8 animaux de chaque groupe ± écart type).

**Tableau III**

| Lot | J0 | J7 | J14 | J20 | J21 | J22 | J23 | J24 |
|---|---|---|---|---|---|---|---|---|
| 1 | 140,5 ± 7,4 | 199,6 ± 13,6 | 259,0 ± 19,6 | 308,2 ± 14,9 | 275,3 ± 15,4 | 301,5 ± 15,8 | 306,6 ± 15,8 | 316,6 ± 16,6 |
| 2 | 141,3 ± 4,9 | 204,1 ± 14,2 | 263,3 ± 14,1 | 313,2 ± 10,9 | 294,4 ± 45,4 | 301,7 ± 12,7 | 310,7 ± 13 | 320,9 ± 12,1 |
| 3 | 139,0 ± 7,1 | 199,2 ± 14,5 | 257 ± 17,7 | 312,6 ± 19,9 | 280,8 ± 18,8 | 310,1 ± 18,5 | 316,9 ± 18,6 | 327,1 ± 20,9 |
| 4 | 138,9 ± 4,6 | 199,1 ± 11,5 | 258,9 ± 17,2 | 305,6 ± 17,8 | 274,5 ± 16,7 | 299,2 ± 15,0 | 305,1 ± 16,0 | 315,3 ± 16,0 |
| 5 | 140,2 ± 10,3 | 200,6 ± 13,4 | 259,4 ± 14,5 | 305,8 ± 15,4 | 274,7 ± 14,8 | 275,9 ± 14,4 | 270,0 ± 13,0 | 276,8 ± 20,9 |
| 6 | 147,2 ± 9,3 | 211,4 ± 19,4 | 270,6 ± 23,8 | 317,3 ± 22,7 | 280,9 ± 22,1 | 288,5 ± 22,0 | 287,4 à 26,6 | 296,6 ± 28,6 |
| 7 | 145,9 ± 14,6 | 208,8 ± 24,2 | 266,1 ± 31,7 | 314,7 ± 34,0 | 281,9 ± 31,2 | 285,4 ± 37,0 | 275,0 ± 38,8 | 286,7 ± 40,7 |
| 8 | 144,6 ± 13,7 | 208,1 ± 19,4 | 266,6 ± 18,2 | 319,1 ± 11,1 | 286,0 ± 13,6 | 297,3 ± 14,3 | 301,8 ± 15,0 | 312,5 ± 12,5 |

L'évolution pondérale montre qu'à J₂₀, tous les animaux ont le même poids. A J₂₁, tous les animaux maigrissent parce que mis à jeun avant l'injection intra-rectale.

A J₂₂, tous les animaux ayant été traités avec une injection intra-rectale de NaCl 9 ‰ reprennent du poids de manière significative.

Le groupe n°8 est le seul ayant reçu une administration déclenchant l'inflammation par le TNBS dont l'évolution pondérale augmente dès J₂₂.

L'évolution pondérale des animaux des groupes 5, 6 et 7 n'augmente modérément qu'à partir de J₂₃ et J₂₄.

La courbe de poids des animaux du groupe 8 étant identique à celle des animaux n'ayant pas reçu de TNBS indique que ces animaux ont recommencé à s'alimenter dès J₂₁. Ces animaux ont donc été protégés contre l'inflammation nécrosante induite par le TNBS.

**Tableau IV**

| Lot | Scores de Wallace | Poids des côlons vidés (g) | Activités MPO (Unités d'absorbance/min/g) |
|---|---|---|---|
| 1 | 0,0 ± 0,0 | 2,04 ± 0,30 | 0,126 ± 0,072 |
| 2 | 0,0 ± 0,0 | 2,19 ± 0,21 | 0,119 ± 0,084 |
| 3 | 0,0 ± 0,0 | 1,95 ± 0,39 | 0,154 ± 0,120 |
| 4 | 0,0 ± 0,0 | 1,96 ± 0,26 | 0,096 ± 0,047 |
| 5 | 5,8 ± 1,0 | 2,82 ± 0,48 | 3,152 ± 1,244 |
| 6 | 5,9 ± 2,0 | 2,90 ± 0,43 | 2,908 ± 1,330 |
| 7 | 6,5 ± 1,9 | 2,99 ± 0,73 | 2,685 ± 0,650 |
| 8 | 3,9 ± 2,7 | 2,76 ± 0,37 | 2,114 ± 1,639 |

Ce résultat est confirmé par la détermination du score de Wallace. En effet, les animaux ayant reçu les compositions conformes à l'invention avec drêches et du TNBS ont un score de Wallace de 3,9, à comparer aux scores moyens de 5,8, 5,9 et 6,5 obtenus pour les autres groupes ayant reçus du TNBS.

Ce score moyen de 3,9 indique un niveau d'inflammation significativement plus faible pour les animaux du groupe 8.

Les résultats des mesures du poids des colons montrent tout d'abord que les animaux ayant reçu une injection de TNBS présentent un côlon de poids supérieur au poids du côlon des animaux qui n'ont pas été traités par le TNBS.

Ce phénomène est notamment dû à l'oedème qui envahit la muqueuse des côlons enflammés.

Le poids moyen du côlon des animaux du groupe 8 reste élevé par rapport au poids moyen des côlons des animaux des groupes n'ayant pas reçu de TNBS, mais il reste le poids le plus faible de tous ceux des groupes d'animaux traités au TNBS.

Quant aux mesures d'activités MPO, à l'évidence faible pour les animaux des groupes non traités au TNBS, il apparaît encore une fois que c'est le groupe 8 qui présente le moins d'activités MPO par rapport aux autres groupes d'animaux traités au TNBS.

Les animaux du groupe 8 sont donc significativement protégés contre l'inflammation nécrosante induite par le TNBS.

### Exemple 2

On étudie chez le rat de laboratoire l'effet des maltodextrines branchées de l'invention (identiques à celles de l'exemple 1) et du dextrose (témoin) sur l'irritation du côlon induite par l'administration de TNBS chez des rats mâles WISTAR et sur leurs performances cognitives dans le test de conditionnement d'évitement d'un stimulus lumineux aversif.

Ce test utilise l'aversion du rat à un environnement fortement éclairé. Le principe est qu'un animal qui souffre est un animal qui apprend moins vite dans le cadre d'un test de conditionnement.

Dans un premier temps, le rat apprend à maîtriser son environnement lumineux aversif dans le cadre d'un conditionnement opérant : l'animal apprend à appuyer sur un levier actif (LA) pour obtenir des périodes d'obscurité de 30 secondes comme renforcement positif.

Le dispositif comprend également un autre levier qui lorsqu'il est actionné ne permet pas d'obtenir de lumière : levier inactif (LI).

Le nombre total d'appuis actifs et inactifs permet d'évaluer le niveau de l'activité manipulatoire des rats.

L'acquisition de l'apprentissage (discrimination entre les deux leviers) est évaluée en comparant les nombres d'appuis sur chacun des deux leviers en phase « lumière » (LA vs LI).

48 rats mâles WISTAR / AF EOPS sont répartis en 4 groupes qui reçoivent dans leur alimentation un régime constitué de la façon décrite dans le tableau V suivant.

**Tableau V**

| Groupe | Régime alimentaire | Nature du traitement à J₁₇ |
|---|---|---|
| 1 | Dextrose (5 %) | Ethanol (20 %) |
| 2 | Dextrose (5 %) | TNBS - alcool (20 %) |
| 3 | MDB (5 %) | Ethanol (20 %) |
| 4 | MDB (5 %) | TNBS - alcool (20 %) |

Après une période de quarantaine au cours de laquelle les animaux reçoivent une alimentation standard et de l'eau potable, les animaux consomment une nourriture additionnée soit de 5 % de maltodextrines branchées de l'invention, soit de 5 % de dextrose durant 15 jours.

A J₁₅, les animaux sont mis à jeun durant 48 heures.

A J₁₇, les rats sont anesthésiés et reçoivent pour 2 groupes sur 4 (groupes 2 et 4) une administration intracolique de 500 µl de TNBS-éthanol 20 % Gay Lussac, à raison de 3 mg/kg de poids corporel (soit 1 mg par rat - cette dose est reconnue pour induire de la douleur associée à une faible irritation intestinale).

De J₁₇ à J₂₃, les animaux continuent à recevoir les régimes complémentés aux MDB ou au dextrose.

A J₂₂, un test cognitif est réalisé : le Test d'Evitement d'un Stimulus Lumineux Aversif (TESLA).

Les tableaux VI et VII suivants présentent le résultat du test TESLA appliqué aux animaux des différents groupes. Le tableau VII présente le nombre d'appuis totaux au cours du test.

**Tableau VI**

| Groupes | 1 | 2 | 3 | 4 |
|---|---|---|---|---|
| ANOVA | | | | |
| F(3,41)= 0,77 ; | 39,33 ± | 35,20 ± | 40,08 ± | 53,27 ± |
| N.S. | 8,63 | 9,43 | 7,43 | 9,72 |

Le tableau VII présente le nombre d'appuis sur les LA et les LI.

Quoique les résultats ne soient pas significativement différents, le tableau VII montre que les rats ayant reçu dans leur alimentation la MDB de l'invention appuient davantage sur les leviers, et plus particulièrement le groupe 4 par rapport au groupe 2.

**Tableau VII**

| Groupes | 1 | 2 | 3 | 4 |
|---|---|---|---|---|
| Appuis LA | 10,50 ± 1,79 | 9,60 ± 2,13 | 10,25 ± 1,45 | 12,45 ± 1,67 |
| Appuis LI | 9,00 ± 2,20 | 8,20 ± 2,02 | 6,42 ± 1,19 | 7,64 ± 1,07 |
| Test t apparié | t = 0,73 | t = 1,63 | t = 4,60 | t = 5,59 |
| (prob. bilat.) | | | | |
| (LA vs LI) | N.S. | N.S. | P < 0,001 | P < 0,001 |
| Signicativité | | | | |

D'après le tableau VIII, seuls les animaux ayant reçu la MDB de l'invention, avec ou sans TNBS, sont capables de faire la différence entre le LA et le LI, en appuyant plus de manière significative sur le LA, démontrant ainsi un effet positif du produit contre la douleur induite par le TNBS.

A J₂₃, les animaux sont sacrifiés ; le côlon est prélevé, examiné selon l'échelle de scores présentée par le tableau VIII suivant.

**Tableau VIII**

| Score des côlons | Observation microscopique |
|---|---|
| 0 | Pas de dommage |
| 1 | Hyperémie localisée sans ulcère |
| 2 | Ulcération sans inflammation significative |
| 3 | Ulcération avec inflammation |
| 4 | Plusieurs sites d'ulcères et d'inflammations ; Taille des ulcères < 1 cm |
| 5 | Multiples sites d'ulcères et d'inflammations ; Taille des ulcères ≥ 1 cm |

Les côlons prélevés sur les 4 groupes sont fixés dans le liquide fixateur de Carson et une observation microscopique est réalisée.

Le tableau IX suivant présente le score des côlons desdits différents groupes.

**Tableau IX**

| Groupes | 1 | 2 | 3 | 4 |
|---|---|---|---|---|
| ANOVA | | | | |
| F(3,41)= 3,89 ; | 2,50 ± 0,42 | 2,10 ± 0,41 | 1,17 ± 0,30 | 1,00 ± 0,36 |
| P = 0,02 | | | | |

L'analyse statistique (ANOVA) montre que les scores des côlons du groupe 3 sont significativement inférieurs à ceux des rats du groupe 1 et tendent à être significativement inférieurs à ceux du groupe 2.

Les scores des côlons des rats du groupe 4 sont également significativement inférieurs à ceux du groupe 1 et tendent à être significativement inférieurs à ceux du groupe 2.

Le tableau X suivant présente le résultat des examens en microscopie réalisés sur les côlons fixés dans le liquide fixateur de Carson (scores macroscopiques), exprimé en terme de degré moyen d'entéropathie (inflammations et nécroses/ulcérations) .

**Tableau X**

| Groupe | | 1 | 2 | 3 | 4 |
|---|---|---|---|---|---|
| Inflammation | Incidence | 12/12 | 12/12 | 12/12 | 12/12 |
| | Degré moyen | 2,8 | 2,8 | 2,3 | 2,2 |
| Nécroses / ulcération | Incidence | 11/12 | 9/212 | 12/12 | 12/12 |
| | Degré moyen | 2,9 | 3,1 | 2,2 | 1,7 |
| Inflammation + | Incidence | 12/12 | 12/12 | 12/12 | 12/12 |
| Nécrose/ulcérations | | | | | |
| | Degré moyen | 2,8 | 2,8 | 2,2 | 1,9 |

Les scores macroscopiques du tableau IX sont moins élevés quant les animaux reçoivent les MDB selon l'invention par rapport aux animaux ayant reçu le dextrose (animaux témoins).

Cette observation est bien corrélée avec l'observation microscopique, car le score de 1,9 a été attribué au groupe 4, tandis qu'il est de 2,8 pour le groupe 2.

On peut donc conclure que tous les animaux présentent une inflammation mais avec des degrés de sévérité différents.

L'entéropathie est moins sévère quand les animaux ont reçu les MDB selon l'invention, ce qui confirme les résultats macroscopiques précédemment énoncés.

Ces résultats sont à mettre en relation avec les résultats du test d'apprentissage démontrant que les animaux qui apprenaient mieux étaient protégés contre la douleur induite par le TNBS démontrant le caractère analgésique de la composition selon l'invention.

### Exemple 3

On étudie l'effet protecteur des maltodextrines branchées de l'invention (celles de l'exemple 1) contre l'inflammation intestinale du porcelet, par le dosage de l'haptoglobine sanguine.

L'haptoglobine est une glycoprotéine plasmatique (α2 globuline) synthétisée par le foie, capable de fixer l'hémoglobine. La teneur en haptoglobine est mesurée par méthode immunologique à l'aide de kits de diagnostic accessibles à l'homme du métier.

La teneur sanguine en haptoglobine augmente dans les syndromes inflammatoires, quelle qu'en soit la cause. Sa cinétique est lente, de sorte que son élévation témoigne d'une certaine ancienneté de l'inflammation.

Au contraire, sa diminution dans le sang traduit un effet protecteur de l'inflammation.

L'essai est effectué sur 1 groupe de 128 porcelets sevrés d'un poids de 7,2 ± 1,04 kg au début de l'étude, groupe divisé en 4 lots de 32 animaux chacun, homogène en poids vif et de sexe (16 mâles castrés et 16 femelles).

Les traitements expérimentaux sont les suivants (pour une période totale de 77 jours) :
Lot n°1 : animaux témoins nourris à l'aide d'une alimentation conventionnelle,
Lot n°2 : animaux recevant de la MDB selon l'invention à raison de 2 % en poids de l'aliment,
Lot n°3 : animaux traités médicalement car nourris avec un aliment contenant deux antibiotiques (chlorotétracycline et spiramycine) à raison de 1000 et 400 mg/kg respectivement, pendant la période d'essai (14 j) puis nourris à nouveau avec une alimentation conventionnelle pendant le délai restant de l'essai (15 - 77 jours),
Lot n°4 : animaux traités médicalement car nourris avec un aliment contenant deux antibiotiques (chlorotétracycline et spiramycine) à raison de 1000 et 400 mg/kg respectivement, pendant la période d'essai (14 j) puis recevant de la MDB selon l'invention à raison de 2 % en poids de l'aliment pendant le délai restant de l'essai (15 - 77 jours).

A la fin de l'essai, on effectue des prélèvements sanguins sur 6 porcelets par sous-groupe et en détermine la teneur en haptoglobine (exprimée en mg/ml de sang).

Le tableau XI suivant présente les résultats obtenus.

**Tableau XI**

| | Lot n°1 | Lot n°2 | Lot n°3 | Lot n°4 |
|---|---|---|---|---|
| Teneur en haptoglobine | 5,74 | 1,83 | 4,45 | 4,36 |

Les résultats montrent que la teneur en haptoglobine du sang des animaux nourris avec la composition anti-inflammatoire et analgésique de l'intestin enrichie en fibres conforme à l'invention (lot n°2) est significativement plus basse que celle des animaux ayant reçu une alimentation conventionnelle, ce qui traduit ainsi un niveau d'inflammation sanguin, donc systémique, plus faible que les témoins.

Dans le lot n°4, le résultat est inférieur à celui du groupe contrôle, même si non significatif. Cette diminution conforte cependant l'effet recherché, à savoir un statut anti-inflammatoire inférieur.

### Exemple 4

On étudie l'effet des maltodextrines branchées de l'invention (celles de l'exemple 1) sur les fermentations intestinales chez le rat de laboratoire.

40 rats OFA d'origine Sprague Dawley sont répartis en 4 groupes qui reçoivent dans leur alimentation un régime alimentaire dont le détail figure dans le tableau XII suivant.

Le groupe 4 reçoit une alimentation complémentée par des fructo-oligosaccharides (RAFTILOSE^{®} P95 commercialisé par la société ORAFTI).

**Tableau XII**

| Lot | Aliment et produit testé |
|---|---|
| 1 | Aliment AO4C |
| 2 | Aliment AO4C + 10 % glucose |
| 3 | Aliment AO4C + 10 % MDB |
| 4 | Aliment AO4C + 10 % RAPTILOSE^{®} P95 |

Après une semaine d'isolement au cours de laquelle les animaux reçoivent une alimentation standard et de l'eau potable, les rats consomment la nourriture durant 36 jours.

A J₀, les animaux sont mis à jeun 24 heures. La boisson est donnée *ad libitum.* A J₁, les fèces sont recueillis.

Le régime décrit du tableau XII est donné aux animaux.

A J₂₈, les animaux sont mis à jeun 24 h. La boisson est donnée *ad libitum.*

A J₂₉, les fèces sont à nouveau recueillies.

A J₃₆, les animaux sont sacrifiés.

Une observation macroscopique générale des organes est réalisée. Les cæcum sont ligaturés et prélevés. Les cæcum pleins, les contenus cæcaux et les cæcum vides sont pesés.

Le pH et la matière sèche des fèces et des contenus cæcaux sont déterminés.

Les activités enzymatiques des fèces sont évaluées également (α-glucosidase et β-glucosidase).

La répartition des acides gras volatils est étudiée au niveau du contenu cæcal (acide acétique, acide propionique, acide butyrique).

Le tableau XIII suivant regroupe les données concernant le poids des cæcums pleins, le poids des cæcums vides, et le pH du contenu cæcal (exprimés en valeur moyenne sur 10 animaux par lot ± écart type).

**Tableau XIII**

| Lot | Poids du cæcum Plein (g) | Poids du cæcum Vide (g) | PH du Contenu cæcal |
|---|---|---|---|
| 1 | 5,64 ± 0,31 | 0,96 ± 0,09 | 6,77 ± 0,28 |
| 2 | 5,78 ± 0,78 | 0,93 ± 0,11 | 6,75 ± 0,18 |
| 3 | 8,29 ± 1,45 | 1,25 ± 0,16 | 6,21 ± 0,16 |
| 4 | 8,68 ± 1,08 | 1,44 ± 0,21 | 6,49 ± 0,20 |

Le tableau XIII montre que le poids des caecum pleins et vides sont significativement plus importants pour les animaux recevant 10 % de MDB selon l'invention ou 10 % de RAFTILOSE^{®} P95 en comparaison des animaux recevant une alimentation standard ou contenant 10 % de dextrose.

Par rapport au lot témoin, le poids du cæcum plein des animaux recevant 10 % de MDB augmente de 46 %, et de 53 % pour les animaux recevant 10 % de RAFTILOSE^{®} P95.

Le poids du cæcum vide évolue quant à lui de 30 % pour le lot recevant de la MDB et de 50 % pour le lot recevant du RAFTILOSE^{®} P95.

Ces résultats montrent que la MDB et le RAFTILOSE^{®} P95 augmentent le poids du caecum et donc de la masse bactérienne cæcale ainsi que le poids de la muqueuse cæcale, entraînant ainsi une protection physique face à une inflammation.

Ce tableau montre également qu'il y a une diminution significative du pH du contenu cæcal pour le lot recevant de la MDB, traduisant ainsi une activité fermentaire cæcale importante.

Cette diminution de pH traduit une augmentation de molécules acides en faveur d'une diminution de molécules basiques qui présenteraient un caractère plus agressif.

Quant à lui, le RAFTILOSE^{®} P95 ne présente pas ces propriétés, puisque le pH du contenu cæcal ne diminue pas de manière significative.

Le tableau XIV regroupe les données relatives à la répartition des acides gras volatils du contenu cæcal.

**Tableau XIV**

| Lot | Acide acétique (mg/g du contenu cæcal) | Acide butyrique (mg/g du contenu cæcal) | Acide propionique (mg/g du contenu cæcal) |
|---|---|---|---|
| 1 | 3,07 ± 0,68 | 2,33 ± 0,97 | 0,75 ± 0,11 |
| 2 | 2,88 ± 0,60 | 1,97 ± 0,63 | 0,76 ± 0,19 |
| 3 | 3,55 ± 0,50 | 2,65 ± 0,67 | 1,56 ± 0,32 |
| 4 | 3,37 ± 0,31 | 2,46 ± 0,56 | 0,99 ± 0,15 |

Ce tableau montre qu'une alimentation supplémentée par 10 % de MDB entraîne une augmentation significative de l'acide propionique du contenu cæcal.

Ce résultat est également obtenu pour le lot recevant du RAFTILOSE^{®} P95, mais de façon moins accentuée.

Aucune différence significative n'apparaît pour le dosage de l'acide acétique du contenu cæcal.

Le tableau XV regroupe les données relatives au pH fécal.

**Tableau XV**

| Lot | J₁ pH des fèces | J₂₉ pH des fèces |
|---|---|---|
| 1 | 6,38 ± 0,34 | 6,58 ± 0,40 |
| 2 | 6,34 ± 0,34 | 6,59 ± 0,29 |
| 3 | 6,37 ± 0,22 | 6,23 ± 0,47 |
| 4 | 6,29 ± 0,40 | 6,23 ± 0,47 |

Ces résultats ne permettent pas de constater une diminution significative du pH fécal chez les animaux recevant de la MDB bien qu'une diminution du pH cæcal ait été observé.

Par contre, le pH fécal diminue pour les animaux recevant du RAFTILOSE^{®} P95.

Les tableaux XVI et XVII regroupent les activités enzymatiques des fèces déterminées respectivement à J₀ et à J₂₉.

**Tableau XVI**

| Lot | α-glucosidase (Uabs/min/g de fèces) | β-glucosidase (Uabs/min/g de fèces) |
|---|---|---|
| 1 | 3,23 ± 1,17 | 4,40 ± 2,86 |
| 2 | 3,19 ± 1,72 | 3,86 ± 2,03 |
| 3 | 3,37 ± 1,85 | 2,55 ± 1,11 |
| 4 | 3,10 ± 1,37 | 2,94 ± 1,19 |

A J₀, il n'y a bien sûr pas de différence significative observée entre les lots.

**Tableau XVII**

| Lot | α-glucosidase (Uabs/min/g de fèces) | β-glucosidase (Uabs/min/g de fèces) |
|---|---|---|
| 1 | 5,62 ± 1,24 | 6,08 ± 1,39 |
| 2 | 5,97 ± 2,60 | 6,74 ± 3,38 |
| 3 | 23,09 ± 7,29 | 24,21 ± 9,10 |
| 4 | 15,32 ± 3,91 | 9,94 ± 3,05 |

A J₂₉, les activités glucosidases sont très fortement augmentées par l'administration de 10 % de MDB. C'est également le cas pour les animaux recevant 10 % de RAFTILOSE^{®} P95, mais de manière moins accentuée.

En effet, des augmentations de 310 % et de 298 % sont observées pour respectivement 1' α-glucosidase et la β-glucosidase du lot recevant de la MDB par rapport au lot témoin, alors que les augmentations ne sont respectivement que de 172 et 63 % pour le lot RAFTILOSE^{®} P95.

Les élévations des activités glucosidases des fèces entraînent la digestion colique des résidus polysaccharidiques présents.

Cette activité glucosidasique élevée peut ainsi entraîner l'augmentation de la bio-disponibilité de certains polyphénols (acteurs importants de la réparation d'une inflammation colique), et aussi la diminution du stress oxydatif.

### Exemple 5

On étudie l'effet des maltodextrines branchées de l'invention (identiques à celles de l'exemple 1) sur la production d'acide butyrique chez le rat de laboratoire.

18 rats de laboratoire FISCHER sont répartis en 3 groupes qui reçoivent dans leur alimentation un régime présenté dans le tableau XVIII suivant.

Le groupe 3 reçoit une alimentation complémentée par des fructo-oligosaccharides (ACTILIGHT^{®} commercialisé par la société BEGHIN-MEIJI).

**Tableau XVIII**

| Lot | Aliment et produit testé |
|---|---|
| 1 | Aliment AO4C |
| 2 | Aliment AO4C + 5 % MDB |
| 3 | Aliment AO4C + 5% d'ACTILIGHT^{®} |

Après une semaine de quarantaine au cours de laquelle les animaux reçoivent une alimentation standard et de l'eau potable, les rats consomment la nourriture énoncée dans le tableau XVIII durant 14 jours.

A J₁₄, les animaux sont sacrifiés. Une observation macroscopique générale des organes est réalisée. Les cæcums sont ligaturés et prélevés.

La répartition des acides gras volatils du contenu cæcal est étudiée.

Le tableau XIX suivant présente les résultats obtenus pour l'acide butyrique.

**Tableau XIX**

| Lot | Acide butyrique (mg/caecum) |
|---|---|
| 1 | 12,6 ± 2,5 |
| 2 | 17,5 ± 3,0 |
| 3 | 16,3 ± 3,8 |

La quantité d'acide butyrique cæcal augmente pour les animaux ayant reçu de la MDB qui peut être classée ainsi parmi les substrats glucidiques butyrogènes chez l'animal.

L'acide butyrique est un facteur important de croissance et de différenciation cellulaire, ce qui justifie l'action de protection de la MDB contre les inflammations du côlon.

### Exemple 6

On étudie chez la souris de laboratoire l'effet des maltodextrines branchées de l'invention (identiques à celles de l'exemple 1) et du dextrose (témoin) sur la production de divers récepteurs cellulaires impliqués dans l'inflammation et l'analgésie de l'intestin chez la souris de laboratoire.

20 souris BALb/c de 7 semaines sont réparties en 2 groupes.

Un groupe reçoit de la boisson consistant en une solution de 10 % de dextrose dans de l'eau potable, l'autre groupe reçoit une solution de 10 % de MDB dans de l'eau potable.

Les animaux reçoivent cette boisson et de l'aliment pour souris standard à volonté pendant 29 jours.

A J₂₉, les souris sont sacrifiées et le côlon est prélevé, sur lequel l'analyse des marqueurs suivants est réalisés :
- « Peroxisome Proliferator-activated receptors » (PPARγ)
- « µ Opioid receptor » (MOR)

Pour évaluer le rôle des maltodextrines branchées de l'invention dans la régulation physiologique de l'inflammation, on isole les ARN totaux des côlons prélevés à l'aide du kit NucleoSpin^{®} RNA II commercialisé par la société CLONTECH LABORATORIES Inc. Les ARN totaux sont retranscrits par la reverse transcriptase en ADNc.

La réaction de transcription réverse est amplifiée et quantifiée par PCR en temps réel (APPLIED BIOSYSTEM) utilisant une amorce pour PPARγ et MOR. Les résultats sont exprimés en nombres de molécules d'ARNm par molécule d'ARNm du contrôle interne de β-actine.

Le tableau XX suivant présente les moyennes des résultats obtenus suite au dosage des PPARγ et des MOR dans la muqueuse colique des rats.

**Tableau XX**

| PPARγ | | MOR | |
|---|---|---|---|
| Lot témoin 10% Dextrose (n=6) | Lot traité 10% MDB (n=8) | Lot témoin 10% Dextrose (n=7) | Lot traité 10% MDB (n=9) |
| 5,02±1,65 | 8,64±2,42 | 0,98±0,70 | 4,34±3,02 |

On observe une augmentation importante de ces deux facteurs avec l'introduction dans l'aliment de 10% de la MDB de la présente invention pendant 29 jours :
- augmentation d'un facteur de 1,72 pour les PPARγ
- augmentation d'un facteur de 4,43 pour les MOR

Les résultats du lot traité à 10% de la MDB sont significativement plus importants que les résultats obtenus pour le lot témoin à 10% de dextrose (p<0,03 pour les PPARγ et p<0,04 pour les MOR).

La MDB de l'invention peut ainsi être une aide à la régulation d'une possible inflammation par le maintien, notamment, de l'intégrité de la muqueuse intestinale.

En effet, l'augmentation du nombre de molécules PPARγ indique que le côlon dispose d'un meilleur statut anti-inflammatoire quand les animaux ont consommé de la MDB.

L'augmentation du nombre de récepteurs à la douleur MOR est quant à elle synonyme d'une diminution de la sensibilité viscérale à la douleur. Ces résultats sont en parfaite adéquation avec ceux de l'exemple 2 dans lequel le rôle analgésique de la MDB avait été démontré par un effet sur le comportement cognitif des animaux.

## Revendications

1. Maltodextrines branchées présentant entre 15 et 35 % de liaisons glucosidiques 1→6, une teneur en sucres réducteurs inférieure à 20 %, un indice de polymolécularité inférieur à 5 et une masse moléculaire en nombre Mn au plus égale à 4500 g/mole pour leur utilisation dans une méthode pour traiter ou prévenir les inflammations de l'intestin et/ou calmer les douleurs au niveau de l'intestin.

2. Maltodextrines branchées selon la revendication 1, **caractérisée en ce que** lesdites maltodextrines branchées présentent une teneur en sucres réducteurs comprise entre 2 et 5 %, et une masse moléculaire moyenne en nombre Mn comprise entre 2000 et 3000 g/mole.

3. Maltodextrines branchées selon l'une ou l'autre des revendications 1 et 2, **caractérisée en ce que** les maltodextrines branchées présentent un taux de fibres totales, déterminé selon la méthode AOAC n°2001-03, supérieur à 50 % sur matière sèche.

4. Composition enrichie en fibres pour une utilisation dans une méthode pour traiter ou prévenir les inflammations de l'intestin et/ou calmer les douleurs au niveau de l'intestin, **caractérisée en ce qu'**elle comprend à titre de principe actif les maltodextrines branchées selon l'une quelconque des revendications 1 à 3.

5. Composition selon la revendication 4, **caractérisée en ce qu'**elle comprend 0,5 à 20 %, de préférence 5 à 10 % en poids sec desdites maltodextrines branchées.

6. Composition selon l'une ou l'autre des revendications 4 et 5 **caractérisée en ce qu'**elle se présente sous forme de boisson, de soupe, de yaourt ou est incorporée dans des céréales du petit déjeuner.

7. Composition selon l'une ou l'autre des revendications 4 et 5, **caractérisée en ce qu'**elle comprend en outre au moins un principe actif choisi dans le groupe constitué par la sulfasalazine et ses dérivés et les corticoïdes.

8. Composition selon l'une ou l'autre des revendications 4 et 5, comprenant en outre un principe actif choisi dans le groupe constitué par la sulfasalazine et ses dérivés, **caractérisée en ce que** le rapport poids de maltodextrines branchées sur poids de sulfasalazine ou d'un de ses dérivés est compris entre 2 et 30.

9. Composition selon l'une ou l'autre des revendications 4 et 5, comprenant en outre un principe actif choisi dans le groupe constitué par les corticoïdes, **caractérisée en ce que** le rapport poids de maltodextines branchées sur poids de corticoïde est compris entre 2 et 250.

10. Composition selon l'une quelconque des revendications 7 à 9 **caractérisée en ce qu'**elle se présente sous forme liquide, en poudre, en sirop, en suppositoire, en comprimé ou en tablette.

11. Kit pour le traitement thérapeutique du corps humain ou animal comprenant :
a) une première composition selon l'une quelconque des revendications 4 à 10 ; et
b) une deuxième composition comprenant un agent anti-inflammatoire de l'intestin.

12. Utilisation de maltodextrines branchés telles que décrites à l'une quelconque des revendications 1 à 3 pour la fabrication d'une composition ou d'un kit pour traiter ou prévenir les inflammations de l'intestin et/ou calmer les douleurs au niveau de l'intestin.

13. Utilisation selon la revendication 12 pour la fabrication d'une composition ou d'un kit pour traiter ou prévenir la maladie inflammatoire chronique de l'intestin, le syndrome de l'intestin irritable, la tourista ou les douleurs abdominales.

14. Utilisation selon la revendication 13 **caractérisée en ce que** la maladie inflammatoire chronique de l'intestin est choisie parmi la recto-colite ulcéro-hémorragique et la maladie de Crohn.

## Claims

1. Branched maltodextrins having between 15% and 35% of 1 → 6 glucosidic linkages, a reducing sugar content of less than 20%, a polymolecularity index of less than 5, and a number-average molecular mass Mn at most equal to 4500 g/mol, for their use in a method for treating or preventing bowel inflammations and/or calming bowel pain.

2. The branched maltodextrin as claimed in claim 1, **characterized in that** said branched maltodextrin has a reducing sugar content of between 2% and 5%, and a number-average molecular mass Mn of between 2000 and 3000 g/mol.

3. The branched maltodextrin as claimed in either of claims 1 and 2, **characterized in that** the branched maltodextrin has a total fiber content, determined according to AOAC method No. 2001-03, of greater than 50% on a dry matter basis.

4. A fiber-enriched composition for use in the use in a method for treating or preventing bowel inflammations and/or calming bowel pain, **characterized in that** it comprises, as active ingredient, the branched maltodextrins as claimed in any one of claims 1 to 3.

5. The composition as claimed in claim 4, **characterized in that** it comprises 0.5% to 20%, preferably 5% to 10% by dry weight of said branched maltodextrins.

6. The composition as claimed in either of claims 4 and 5, **characterized in that** it is in the form of a drink, a soup or a yogurt or is incorporated into breakfast cereals.

7. The composition as claimed in either of claims 4 and 5, **characterized in that** it also comprises at least one active ingredient chosen from the group consisting of sulfasalazine and its derivatives and corticoids.

8. The composition as claimed in either of claims 4 and 5, also comprising an active ingredient chosen from the group consisting of sulfasalazine and its derivatives, **characterized in that** the ratio by weight of branched maltodextrins to weight of sulfasalazine or of one of its derivatives is between 2 and 30.

9. The composition as claimed in either of claims 4 and 5, also comprising an active ingredient chosen from the group consisting of corticoids, **characterized in that** the ratio by weight of branched maltodextrins to weight of corticoids is between 2 and 250.

10. The composition as claimed in any one of claims 7 to 9, **characterized in that** it is in the form of a liquid, a powder, a syrup, a suppository, a tablet or a lozenge.

11. A kit for the therapeutic treatment of the human or animal body, comprising:
a) a first composition as claimed in any one of claims 4 to 10; and
b) a second composition comprising an antiinflammatory agent for the bowel.

12. The use of branched maltodextrins as described in any one of claims 1 to 3, for the manufacture of a composition or of a kit for treating or preventing bowel inflammations and/or calming bowel pain.

13. The use as claimed in claim 12, for the manufacture of a composition or of a kit for treating or preventing chronic inflammatory bowel disease, irritable bowel syndrome, traveler's diarrhea or abdominal pain.

14. The use as claimed in claim 13, **characterized in that** the chronic inflammatory bowel disease is chosen from ulcerative colitis and Crohn's disease.

## Patentansprüche

1. Verzweigte Maltodextrine, die zwischen 15 und 35% 1→6-glycosidische Bindungen, einen Gehalt an reduzierenden Zuckern von kleiner als 20%, einen Polymolekularitätsindex von kleiner als 5 und ein zahlenmittleres Molekulargewicht Mn von höchstens 4500 g/Mol aufweisen, zur Verwendung in einem Verfahren, um Entzündungen des Darms zu behandeln oder zu verhindern und/oder um Schmerzen im Bereich des Darms zu lindern.

2. Verzweigte Maltodextrine gemäß Anspruch 1, **dadurch gekennzeichnet, dass** die verzweigten Maltodextrine einen Gehalt an reduzierenden Zuckern zwischen 2 und 5% und ein zahlenmittleres Molekulargewicht Mn zwischen 2000 und 3000 g/Mol aufweisen.

3. Verzweigte Maltodextrine gemäß einem der Ansprüche 1 oder 2, **dadurch gekennzeichnet, dass** die verzweigten Maltodextrine einen Gesamtfasergehalt, gemessen gemäß dem Verfahren AOAC Nr. 2001-03, von größer als 50%, bezogen auf die Trockensubstanz, aufweisen.

4. Zusammensetzung, die mit Fasern angereichert ist, zur Verwendung in einem Verfahren, um Entzündungen des Darms zu behandeln oder zu verhindern und/oder um Schmerzen im Bereich des Darms zu lindern, **dadurch gekennzeichnet, dass** sie als Wirkstoff verzweigte Maltodextrine gemäß einem der Ansprüche 1 bis 3 umfasst.

5. Zusammensetzung gemäß Anspruch 4, **dadurch gekennzeichnet, dass** sie 0,5 bis 20%, bevorzugt 5 bis 10%, bezogen auf das Trockengewicht dieser verzweigten Maltodextrine, umfasst.

6. Zusammensetzung gemäß einem der Ansprüche 4 oder 5, **dadurch gekennzeichnet, dass** sie in Form eines Getränks, einer Suppe, eines Joghurts vorliegt oder in Frühstückscerealien enthalten ist.

7. Zusammensetzung gemäß einem der Ansprüche 4 oder 5, **dadurch gekennzeichnet, dass** sie weiterhin mindestens einen Wirkstoff umfasst, der aus der Gruppe, bestehend aus Sulfasalazin und seinen Derivaten und Corticoiden, ausgewählt ist.

8. Zusammensetzung gemäß einem der Ansprüche 4 oder 5, weiterhin umfassend einen Wirkstoff, der aus der Gruppe, bestehend aus Sulfasalazin und seinen Derivaten, ausgewählt ist, **dadurch gekennzeichnet, dass** das Gewichtsverhältnis von verzweigten Maltodextrinen zu dem Gewicht von Sulfasalazin oder einem seiner Derivate zwischen 2 und 30 ist.

9. Zusammensetzung gemäß einem der Ansprüche 4 oder 5, weiterhin umfassend einen Wirkstoff, der aus der Gruppe, bestehend aus Corticoiden, ausgewählt ist, **dadurch gekennzeichnet, dass** das Gewichtsverhältnis von verzweigten Maltodextrinen zu dem Gewicht von dem Corticoid zwischen 2 und 250 ist.

10. Zusammensetzung gemäß einem der Ansprüche 7 bis 9, **dadurch**
**gekennzeichnet, dass** sie in flüssiger Form, als Pulver, als Sirup, als Suppositorium, als Tablette oder als Pastille vorliegt.

11. Kit zur therapeutischen Behandlung des menschlichen oder tierischen Körpers, umfassend:
a) eine erste Zusammensetzung gemäß einem der Ansprüche 4 bis 10; und
b) eine zweite Zusammensetzung, umfassend ein entzündungshemmendes Mittel für den Darm.

12. Verwendung von verzweigten Maltodextrinen, wie in einem der Ansprüche 1 bis 3 beschrieben, zur Herstellung einer Zusammensetzung oder eines Kits, um Entzündungen des Darms zu behandeln oder zu verhindern und/oder um Schmerzen im Bereich des Darms zu lindern.

13. Verwendung gemäß Anspruch 12 zur Herstellung einer Zusammensetzung oder eines Kits, um chronisch-entzündliche Darmerkrankung, Reizdarmsyndrom, Reisedurchfall oder Abdominalschmerzen zu behandeln oder zu verhindern.

14. Verwendung gemäß Anspruch 13, **dadurch gekennzeichnet, dass** die chronisch-entzündliche Darmerkrankung aus Colitis ulcerosa und Morbus Crohn ausgewählt ist.
